# EUROPÄISCHE PATENTSCHRIFT

(11) **EP 3 892 868 B1**
(45) Veröffentlichungstag und Bekanntmachung des Hinweises auf die Patenterteilung: **01.03.2023**
(21) Anmeldenummer: 21020184.4
(22) Anmeldetag: 06.04.2021
(51) Int. Cl.: F15D 1/02, A61M 16/08, G01F 1/00, A61M 16/00, A61B 5/087

(54) **GASFLUSSLEITEINRICHTUNG FÜR EIN BEATMUNGSGERÄT ZUR BEATMUNG EINES LEBEWESENS**
GAS FLOW GUIDING DEVICE FOR A VENTILATION APPARATUS FOR VENTILATION OF A LIVING ORGANISM
DISPOSITIF DE GUIDAGE D'ÉCOULEMENT DE GAZ POUR UN APPAREIL RESPIRATOIRE DESTINÉ À LA RESPIRATION D'UN ÊTRE VIVANT

(30) Priorität: 09.04.2020 DE 102020002234
(43) Veröffentlichungstag der Anmeldung: 13.10.2021
(73) Patentinhaber: Löwenstein Medical Technology S.A., 2557 Luxembourg (LU)
(72) Erfinder: Schattner, Jan, 25421 Pinneberg (DE); Göbel, Christof, 22457 Hamburg (DE); Adametz, Benjamin, 22609 Hamburg (DE)
(74) Vertreter: Marx, Thomas

(56) Entgegenhaltungen:
- EP-A1- 2 138 809
- WO-A1-2014/129913
- DE-A1-102017 130 346
- DE-U1-202018 100 533
- US-A1- 2013 098 484
- US-A1- 2014 026 677
- US-A1- 2014 150 798
- US-A1- 2018 243 518
- US-B1- 6 915 705

## Beschreibung

Die Erfindung betrifft eine Gasflussleiteinrichtung zur Verwendung für eine Gasflusssteuerungsvorrichtung und für ein Therapiegerät zur Zuführung von Atemgas zu einem Lebewesen.

Therapie- bzw. Beatmungsgeräte werden unter anderem zur Beatmung eines Lebewesens mit einem Atemgas eingesetzt. Weiter können Therapiegeräte auch für eine gezielte Unterstützung des Hustens eingesetzt werden. Die Beatmung von Patienten in Krankenhäusern oder im häuslichen Umfeld mit einem Atemgas ist ein übliches Anwendungsfeld solcher Beatmungsgeräte. Zur Beatmung der Lebewesen, beispielsweise des Patienten, ist das Atemgas aus Umgebungsluft und Sauerstoffgas herzustellen, d.h. in aller Regel zu mischen.

Zur Beatmung der zu beatmenden Lebewesen ist es erforderlich, ein Atemgasvolumen in einer erforderlichen Frequenz und mit einem erforderlichen Volumen bzw. einer erforderlichen Masse bereitzustellen. Hierzu ist es bekannt, einen Volumenstrom und/oder Massestrom eines Gasflusses zu erfassen.

Soweit in der vorliegenden Patentanmeldung lediglich von dem Volumenstrom oder von dem Massestrom die Rede ist, gelten die Ausführungen zumindest dem Grunde nach sowohl für den Volumenstrom als auch für den Massestrom.

Der Gasfluss umfasst beispielsweise das Atemgas und/oder das Sauerstoffgas und/oder die Umgebungsluft. Zur Erfassung des Volumenstroms werden entsprechende Sensoren in einem Beatmungsgerät, insbesondere in den Gasfluss führenden Elementen, angeordnet.

Ein zu beatmendes Lebewesen wird die Beatmung mit einem Atemgas durch das Beatmungsgerät als natürlich empfinden, wenn das Beatmungsgerät dem zu beatmenden Lebewesen das Atemgas mit dem für das Lebewesen üblichen Atemgasvolumen, mit der üblichen Frequenz und/oder mit dem üblichen Druck bereitstellt. Je genauer eine Messvorrichtung bzw. Sensoren den Volumenstrom erfassen, desto besser lassen sich Volumen, Druck und Frequenz für eine natürlich wirkende Beatmung einstellen. Um einen Volumenstrom mit einer höheren Genauigkeit zu erfassen, ist es bekannt, an einem Einlass der Volumenstrommessvorrichtung ein Beruhigungsgitter anzuordnen. Solch ein Beruhigungsgitter beruhigt den Volumenstrom des zu erfassenden Gasflusses und richtet diesen aus. Ein derart beruhigter Volumenstrom lässt sich mit einer Messvorrichtung sodann wesentlich exakter erfassen.

Es ist bekannt, dass solche Beruhigungsgitter mit zunehmender Beatmungsdauer den Volumenstrom zunehmend aufwirbeln. Denn mit zunehmender Beatmungsdauer verfängt sich in dem Beruhigungsgitter in nachteilhafter Weise Schmutz, Pollen, Staub, Keime oder auch Ausscheidungen des zu beatmenden Lebewesens. Solche Verschmutzungen wirbeln den Volumenstrom des Gasflusses auf und verfälschen Messergebnisse der Messvorrichtung. In der Folge wird die Qualität der Beatmung eines zu beatmenden Lebewesens reduziert. Nachteilig ist ebenfalls, dass die Beatmung nicht als natürlich wahrgenommen wird.

Der zunehmenden Verschmutzung und potenziellen Ansiedelung von Keimen begegnet man üblicherweise durch Austausch der Beruhigungsgitter und sonstiger luft- bzw. gasflussführender Komponenten des Beatmungsgeräts. Der Austausch der Beruhigungsgitter und der sonstigen gasflussführenden Elemente ist ein wesentlicher Kostenfaktor der Beatmung.

Verschmutzte Beruhigungsgitter und luftführende Elemente des Beatmungsgeräts können ebenfalls gereinigt werden. Die Reinigung umfasst insbesondere die Desinfektion und/oder Sterilisation. Für die Desinfektion und/oder Sterilisation ist u.a. der Einsatz von Formaldehyd bekannt. Formaldehyd hat allerdings den Nachteil, dass die zu reinigenden Elemente und insbesondere Schaumstoffe der zu reinigenden Elemente das Formaldehyd absorbieren. Dies erfordert im Anschluss erneut lange Desorptionszeiten, bis die Komponenten wieder eingesetzt werden können, um zu verhindern, dass die zu beatmenden Lebewesen mit dem giftigen Formaldehyd in Kontakt kommen. Ferner ist es bekannt, die Beruhigungsgitter und gasflussführenden Elemente des Beatmungsgeräts mit Wasserstoffperoxid und/oder Ozon zu desinfizieren. Diese Reinigungsverfahren haben den Nachteil, dass eine für die Beatmung von Lebewesen angemessene Desinfektion der Elemente nicht immer bzw. lediglich mit einem hohen Aufwand sichergestellt werden kann. Zusätzlich kommt es durch die Desinfektion nicht zu einer mechanischen Entfernung von Stoffen oder Partikeln am Gitter. Ferner müssen die Beruhigungsgitter und Elemente aus Materialien hergestellt sein, die gegenüber Wasserstoffperoxid und/oder Ozon beständig sind.

Die Dokumente US 6 915 705 B1, US 2018/243518 A1, WO 2014/129913 A1 und US 2014/150798 A1 offenbaren Gasflussleiteinrichtungen (oder Flow-Sensoren) zum Leiten eines Gasflusses in einem Therapiegerät zur Atemgaszufuhr bei Lebewesen. Diese weisen einen Ringkörper und eine innerhalb des Ringkörpers angeordnete Leiteinheit zum Leiten des durch den Ringkörper geführten Gasflusses auf, wobei die Leiteinheit mindestens ein Leitelement umfasst.

Insoweit ist eine Aufgabe der vorliegenden Erfindung, eine kostengünstige Gasflussleiteinrichtung bereitzustellen, welche kontinuierlich auf verlässliche Weise für die Bereitstellung eines beruhigten Volumenstroms für eine Gasflusssteuerungsvorrichtung eines Beatmungsgeräts sorgen kann, insbesondere unabhängig von einer Beatmungsdauer. Weiterhin soll die Problematik der Reinigung bzw. Desinfektion der Komponenten verbessert werden.

Gemäß einem ersten Aspekt der Erfindung wird die Aufgabe mit einer Gasflussleiteinrichtung zum Leiten eines Gasflusses in einer Gasflussmessvorrichtung eines Beatmungsgeräts zur Beatmung eines Lebewesens nach Anspruch 1 gelöst.

Die Gasflussleiteinrichtung zum Leiten eines Gasflusses in einer Gasflussmessvorrichtung umfasst einen Ringkörper und eine Leiteinheit.

Der Ringkörper ist zur Führung des Gasflusses ausgebildet. Der Ringkörper erstreckt sich entlang einer Axialachse mit einer Ringkörperlänge von einem ersten Ringkörperende zu einem zweiten Ringkörperende. Der Ringkörper weist an dem ersten Ringkörperende eine erste Durchgangsöffnung und an dem zweiten Ringkörperende eine zweite Durchgangsöffnung auf. Zwischen der ersten und zweiten Durchgangsöffnung erstreckt sich ein Ringkanal, der innerhalb des Ringkörpers angeordnet ist. Ein Innenmantel des Ringköpers begrenzt den Ringkanal ausgehend von der Axialachse in einer radialen Richtung entlang der Axialachse.

Die Leiteinheit ist zum Leiten des durch den Ringkörper geführten Gasflusses innerhalb des Ringkanals angeordnet. Die Leiteinheit umfasst mindestens ein Leitelement, das sich entlang der Axialachse mit einer ersten Leitelementlänge von einem ersten Leitelementende zu einem zweiten Leitelementende erstreckt. Das mindestens eine Leitelement erstreckt sich in einer radialen Richtung mit einer ersten Leitelementhöhe von einem ersten Leitelementfuß zu einer mindestens ersten Leitelementspitze. Dabei weist das mindestens eine Leitelement beispielhaft zwischen dem ersten Leitelementende und dem zweiten Leitelementende eine Zunahme der Leitelementhöhe bis hin zur Leitelementspitze auf, wobei die Leitelementhöhe an der Leitelementspitze über eine Länge gleichbleiben kann, sich also ein Plateau auf Höhe der Leitelementspitze ausbilden kann. Ferner kann das Leitelement zwei und mehr Leitelementspitzen aufweisen. Die zwei oder mehr Leitelementspitzen können dabei unterschiedliche Höhen aufweisen. Falls das Leitelement zwei oder mehr Leitelementspitzen aufweist, dann ist beispielhaft die erste Leitelementspitze höher als die dahinterliegenden Leitelementspitzen. Das mindestens eine Leitelement weist in Umfangsrichtung eine erste Leitelementdicke auf.

Der Innenmantel weist ausgehend von der Axialachse einen Innenmantelradius auf. Dieser Innenmantelradius ist größer als eine Maximalhöhe der ersten Leitelementhöhe des mindestens einen Leitelements. Es können auch mehrere Leitelemente vorgesehen sein, sodass dann insbesondere der Innenmantelradius größer als die jeweilige Maximalhöhe der ersten Leitelementhöhe aller Leitelemente ist.

Wesentlich ist an der vorliegenden Erfindung, dass bei den Leiteinheiten der Gasflussleiteinrichtungen ein zentraler, offener Bereich bzw. Kanalabschnitt vorgesehen ist, der einen freie Durchgangsöffnung für den Gasfluss ausbildet. Dadurch kann sich im Vergleich zu sich über den gesamten Querschnitt erstreckenden Beruhigungsgittern des Standes der Technik Schmutz nicht mehr so leicht ansammeln und den Strömungsquerschnitt verkleinern oder gar verstopfen. Durch die vorschlagsgemäßen Gasflussleiteinrichtungen ist somit die Gefahr an Verstopfungen im Einsatz deutlich verringert, wodurch für die sogenannte Flowmessstrecke des Beatmungsgerätes kontinuierlich ein beruhigter, unverfälschter Gasfluss bereitgestellt werden kann. Es entstehen damit auch seltener Fehlsignale.

Ein weiterer wesentlicher Aspekt der vorliegenden Erfindung ist die zunächst ansteigende und dann, nach Erreichen einer Maximalhöhe des Leitelements, wieder abfallende radiale Höhe des Leitelements. Hierdurch wird eine ebene Prallfläche vermieden, sodass sich Partikel leichter nach innen und damit durch zentralen, offenen Bereich bzw. Kanalabschnitt bewegen.

Ferner müssen dadurch, dass die Gasflussleiteinrichtungen austauschbar, sprich auch wieder aus einer Gasflussteuerungsvorrichtung entnehmbar und erneut einsetzbar, sind, die Komponenten im Unterschied zu verstopften Beruhigungsgittern des Standes der Technik nicht mehr entsorgt und durch neue ersetzt werden, sondern können vielmehr auf einfachere Weise gereinigt und wiederverwendet werden. Der zentrale, freie Kanalabschnitt, welche durch die Leiteinheiten nicht belegt ist, erleichtert nämlich auch die Reinigung der entsprechenden Gasflussleiteinrichtungen. Sind separat entnehmbare Gitter in der Gasflussleiteinrichtung angeordnet bzw. mit dieser kombiniert, können auch diese entnommen werden und gereinigt werden, statt entsorgt zu werden.

Die Leitelemente der vorschlagsgemäßen Leiteinheit der Gasflussleiteinrichtung dienen als eine Art Finnen, die den Gasfluss in effektiver Weise beruhigen, ohne so schnell zu verstopfen, wie es den gesamten Strömungsquerschnitt blockierende Beruhigungsgitter tun.

Der Ringkanal ist vorzugsweise im Wesentlichen zylinderförmig ausgebildet. Insbesondere ist der Innenmantel zylinderförmig ausgebildet. Vorzugsweise erstreckt sich der Ringkanal und insbesondere der Innenmantel konzentrisch entlang der Axialachse.

In alternativen Ausführungen kann der Ringkanal auch beispielsweise über einen rechteckigen Querschnitt, einen elliptischen Querschnitt oder eine Freiform verfügen.

Der Ringkörper ist insbesondere als Hohlkörper mit einer ersten und zweiten Durchgangsöffnung ausgebildet, wobei der Ringkanal den Hohlraum des Ringkörpers definiert. Vorzugsweise weist der Ringkörper einen Außenmantel auf. Der Außenmantel weist einen Außenmantelradius ausgehend von der Axialachse auf. Zwischen dem Außenmantel und dem Innenmantel erstreckt sich eine Wand des Ringkörpers. Die Wand weist eine Wandstärke, welche sich insbesondere aus der Differenz des Außenmantelradius abzüglich des Innenmantelradius ergibt. Die Wandstärke des Ringkörpers ist beispielhaft im Wesentlichen konstant entlang der Axialachse und in Umfangsrichtung.

Insbesondere ist der Ringkörper zylinderförmig ausgebildet. Vorzugsweise ist der Außenmantel zylinderförmig ausgebildet. Es kann aber auch sein, dass der Außenmantel eine rechteckige Form oder eine Freiform aufweist. Insbesondere ist der Außenmantel des Ringkörpers in Umfangsrichtung entlang der Axialachse im Wesentlichen geschlossen. Der Innenmantel begrenzt den Ringkanal in radialer Richtung entlang der Axialachse. Vorzugsweise ist der Innenmantel zylinderförmig ausgebildet. Es kann sein, dass der Innenmantel eine rechteckige Form oder eine Freiform aufweist. Ferner kann sein, dass der Außenmantel und/oder der Innenmantel kegelförmig ausgebildet sind. Insbesondere können der Außenmantel und/oder der Innenmantel in Umfangsrichtung polygonal ausgebildet sein. Insbesondere kann sein, dass der Innenmantel in Umfangsrichtung polygonal ausgebildet ist.

Die erste und/oder zweite Durchgangsöffnung sind vorzugsweise kreisförmig ausgebildet. Es kann alternativ sein, dass die erste und/oder zweite Durchgangsöffnung elliptisch und/oder polygonal, beispielsweise rechtwinklig, ausgebildet sind.

Insbesondere ist die Gasflussleiteinrichtung zur lösbaren Montage und Demontage ausgebildet. Dadurch ist die Gasflussleiteinrichtung zur einfachen und schnellen Reinigung geeignet.

Insbesondere umfasst die Gasflussleiteinrichtung beispielsweise eines der folgenden Materialen oder eine Kombination daraus: Aluminium, Edelstahl oder Ozon- und/oder Wasserstoffperoxid-beständige Kunststoffe. Solche Kunststoffe sind beispielsweise EPDM, PE, PEEK, PES, POM, HNBR, NBR, PA, PET, PUR, PVC, Silikon etc.

Die Erfindung hat insbesondere den Vorteil, dass Fremdkörper oder Partikel und sowie auch Keime wesentlich seltener in der Gasflussleiteinrichtung verfangen. Diesen Vorteil begründet insbesondere die geometrische Ausbildung der vorschlagsgemäßen Leiteinheit. Die erfindungsgemäße Gasflussleiteinrichtung hat somit insbesondere den Effekt, dass die Gesundheits- und Lebensgefahr zu beatmender Lebewesen wesentlich reduziert wird. Ferner mindert die erfindungsgemäße Gasflussleiteinrichtung Verwirbelungen des zu fördernden Gasflusses. Dies erhöht die Genauigkeit, mit der sich der Volumenstrom erfassen lässt, und steigert in der Folge die Qualität der Therapie erheblich. Insbesondere lässt sich das Beatmungsgerät mit der erfindungsgemäßen Gasflussleiteinrichtung derart einstellen, dass die Beatmung wesentlich natürlicher wirkt.

Die Erfindung hat in einer beispielhaften Ausführungsform ferner den Vorteil, dass sich die Gasflussleiteinrichtung besonders einfach und schnell demontieren und reinigen lässt. Insoweit stellt die Erfindung eine Gasflussleiteinrichtung für eine besonders kostengünstige Beatmung von Lebewesen bereit. Insbesondere lässt sich eine solche Gasflussleiteinrichtung besonders einfach und schnell reinigen.

Gemäß einer ersten beispielhaften Ausführungsform der Gasflussleiteinrichtung weist die Leiteinheit mindestens ein zweites Leitelement auf, das sich entlang der Axialachse mit einer zweiten Leitelementlänge von einem ersten Leitelementende zu einem zweiten Leitelementende erstreckt, sich in einer radialen Richtung mit einer zweiten Leitelementhöhe von einem zweiten Leitelementfuß zu einer zweiten Leitelementspitze erstreckt, und in Umfangsrichtung eine zweite Leitelementdicke aufweist, und in Umfangsrichtung eine zweite Leitelementdicke aufweist, wobei der Innenmantelradius des Innenmantels größer ist als eine Maximalhöhe der zweiten Leitelementhöhe des mindestens einen zweiten Leitelements. Es können auch mehrere zweite Leitelemente vorgesehen sein, sodass dann insbesondere der Innenmantelradius größer als die jeweilige Maximalhöhe der zweiten Leitelementhöhe aller zweiten Leitelemente ist.

Diese beispielhafte Ausführungsform hat den Vorteil, dass sich gemäß dem Anwendungsgebiet die Gasflussleiteinrichtung und insbesondere die Geometrie der Leiteinheit flexibel und kostengünstig einstellen lässt. Der zentrale, für eine Durchströmung freie Kanalabschnitt kann individuell eingestellt werden.

In einer weiteren beispielhaften Fortbildung der Gasflussleiteinrichtung ist das mindestens eine Leitelement mit dem Leitelementfuß an dem Innenmantel des Ringkanals angebracht und erstreckt sich radial in den Ringkanal. Insbesondere erstreckt sich das mindestens erste Leitelement und/oder das mindestens zweite Leitelement radial in Richtung der Axialachse. Bei nicht-kreisförmigen Querschnitten ist radial so zu verstehen, dass die Richtung vom Innenmantel des Ringkanals zur Querschnittsmitte des Ringkanals beschrieben wird. Insbesondere ist gemeint, dass sich das Leitelement vom Innenmantel des Ringkanals in Richtung Querschnittsmitte des Ringkanals erstreckt.

Gemäß der Erfindung ist die Maximalhöhe der ersten Leitelementhöhe des mindestens einen ersten Leitelements größer als die Maximalhöhe der zweiten Leitelementhöhe des mindestens einen zweiten Leitelements. Insbesondere ist bei einer Vielzahl an ersten und zweiten Leitelementen die Maximalhöhe aller ersten Leitelemente größer als die der zweiten Leitelemente. Nach einer weiteren beispielhaften Ausführungsform beträgt die Maximalhöhe der ersten Leitelementhöhe des mindestens einen ersten Leitelements mindestens 50 %, weiter bevorzugt mindestens 75 %, des Innenmantelradius und/oder die Maximalhöhe der zweiten Leitelementhöhe des mindestens einen zweiten Leitelements zwischen 15 % und 75 %, vorzugsweise zwischen 25 % und 50%, des Innenmantelradius.

Nach einer weiteren beispielhaften Fortbildung sind die mindestens einen ersten Leitelemente äquidistant angeordnet und/oder die mindestens einen zweiten Leitelemente äquidistant angeordnet. Insbesondere sind die Leitelemente äquidistant über den Umfang des Innenmantels des Ringkörpers verteilt.

Gemäß einer weiteren beispielhaften Fortbildung weist die Leiteinheit eine erste Anzahl an ersten Leitelementen und eine zweite Anzahl an zweiten Leitelementen auf, wobei die erste Anzahl an ersten Leitelementen und die zweite Anzahl an zweiten Leitelementen gleich sind oder die zweite Anzahl an zweiten Leitelementen größer ist als die erste Anzahl an ersten Leitelementen.

Gemäß einer weiteren beispielhaften Fortbildung der Gasflussleiteinrichtung sind zwischen zwei in Umfangsrichtung benachbart angeordneten ersten Leitelementen ein oder zwei oder mehr als zwei zweite Leitelemente angeordnet.

In einer weiteren beispielhaften Ausführungsform ist die Gasflussleiteinrichtung dadurch gekennzeichnet, dass die erste Leitelementlänge des mindestens einen ersten Leitelements in radialer Richtung entlang der ersten Leitelementhöhe variiert oder konstant ist und/oder die zweite Leitelementlänge des mindestens einen zweiten Leitelements in radialer Richtung entlang der zweiten Leitelementhöhe variiert oder konstant ist; und/oder die erste Leitelementhöhe des mindestens einen ersten Leitelements und/oder die zweite Leitelementhöhe des mindestens einen zweiten Leitelements entlang der Axialachse variiert oder konstant ist; und/oder die erste Leitelementdicke des mindestens einen ersten Leitelements in radialer Richtung entlang der ersten Leitelementhöhe variiert oder konstant ist und/oder die zweite Leitelementdicke des mindestens einen zweiten Leitelements in radialer Richtung entlang der ersten Leitelementhöhe variiert oder konstant ist.

Vorzugsweise ist die Maximallänge der ersten Leitelementlänge größer als die Maximalhöhe der ersten Leitelementhöhe. Ferner kann es sein, dass die Maximalhöhe der ersten Leitelementhöhe größer ist als die Maximalbreite der ersten Leitelementbreite. Insbesondere kann die erste Leitelementhöhe ausgehend von dem ersten Leitelementende zu dem zweiten Leitelementende des ersten Leitelements zunehmen. Insbesondere entspricht die erste Leitelementlänge maximal der Ringkörperlänge. Vorzugsweise ist die erste Leitelementlänge kleiner ist als die Ringkörperlänge. Diese Ausführungen gelten analog für die geometrischen Abmessungen des mindestens zweiten Leitelements.

Das Verhältnis zwischen maximaler Höhe des Leitelements Hmax und maximaler Dicke des Leitelements Dmax beträgt zwischen 50:1 und 2:1.

Das Verhältnis zwischen maximaler Höhe des Leitelements Hmax und maximaler Länge des Leitelements Lmax beträgt zwischen 4:1 und 1:10.

Das Verhältnis zwischen maximaler Dicke des Leitelements Dmax und maximaler Länge des Leitelements Lmax beträgt zwischen 1:125 und 1:2.

Das Verhältnis zwischen dem Innenmantelradius IR zur maximalen Höhe der Leitelemente Hmax beträgt zwischen 10:9 und 10:1.

Das Verhältnis zwischen dem Innenradius IR und dem Radius des durch die Leitelemente begrenzten, zentralen, offenen Bereichs beträgt 10:1 und 10:9.

Das Verhältnis zwischen der maximalen Höhe der Leitelemente Hmax und dem Radius des durch die Leitelemente begrenzten, zentralen, offenen Bereichs beträgt 9:1 und 1:9.

In einer Ausführungsform der Leitelemente beträgt die Länge Lmax im Bereich des Leitelemetfußes 4 mm bis 50 mm. In einer bevorzugten Ausführungsform beträgt die Länge Lmax 12 mm bis 30 mm.

In einer Ausführungsform beträgt die maximale Breite Dmax der Leitelemente 0,4 mm bis 2,5 mm. In einer bevorzugten Ausführungsform beträgt die Wandstärke Dmax 0,8 mm bis 1,8 mm.

In einer Ausführungsform beträgt die maximale Höhe Hmax der Leitelemente 1 mm bis 45 mm

In einer Ausführungsform beträgt der Innenmantelradius IR 5 mm bis 50 mm.

Gemäß einer weiteren beispielhaften Ausführungsform ist die Leiteinheit einstückig mit dem Ringkörper ausgebildet oder ist in den Ringkanal des Ringkörpers eingesetzt.

Bei einer einstückig ausgebildeten Gasflussleiteinrichtung sind die Leiteinheit und der Ringkörper insbesondere aus einem Stück hergestellt.

Gemäß einer weiteren beispielhaften Ausführungsform ist die einstückig mit dem Ringkörper ausgebildete Leiteinheit spanend und/oder gießend und/oder fügend erzeugt.

Insbesondere sind bei einer einstückig ausgebildeten Gasflussleiteinrichtung aus einem Stück zerspanend hergestellt und/oder aus einem Stück gießend, insbesondere spritzgießend, hergestellt und/oder fügend, insbesondere mit einer Schweißverbindung, miteinander verbunden.

Bei einer nicht einstückig ausgebildeten Gasflussleiteinrichtung ist die Leiteinheit insbesondere kraft- und/oder stoffschlüssig an bzw. in dem Ringkörper angeordnet bzw. angebracht. Insbesondere kann bei einer nicht einstückig ausgebildeten Gasflussleiteinrichtung die Leiteinheit beispielsweise auch mit dem Ringkörper verklebt sein.

Gemäß einer weiteren beispielhaften Fortbildung weist der Ringkörper ausgehend von dem ersten Ringkörperende einen ersten Ringkörperendabschnitt und ausgehend von dem zweiten Ringkörperende einen zweiten Ringkörperendabschnitt auf, zwischen denen sich ein Ringkörpermittelabschnitt erstreckt, wobei der erste und/oder zweite Ringkörperendabschnitt über den Umfang verteilt wenigstens eine sich von dem Außenmantel bis zu dem Innenmantel des Ringkörpers erstreckende Bohrung oder Aussparung aufweist zur Weiterleitung pneumatischer Drucksignale an vorgesehene und außerhalb der Leiteinrichtung angeordnete Sensoren, wobei die mindestens eine Aussparung vorzugsweise zu dem ersten und/oder zweiten Ringkörperende offen, insbesondere als Nut, ausgebildet ist.

Die Aufgabe wird ferner gemäß einem zweiten Aspekt der Erfindung mit einer Gasflusssteuerungsvorrichtung gelöst.

Die Gasflusssteuerungsvorrichtung umfasst mindestens eine zuvor beschriebene Gasflussleiteinrichtung nach dem ersten Aspekt der Erfindung sowie eine Steuervorrichtung zur Steuerung eines Gasflusses, insbesondere zur Regelung des Gasflusses. Beispielsweose ist weiterhin eine Messvorrichtung zur Erfassung des Gasflusses vorgesehen.

Insbesondere ist die Gasflussleiteinrichtung demontierbar in der Gasflusssteuerungsvorrichtung angeordnet.

Vorzugsweise kann die Gasflussleiteinrichtung auf einfache Weise in der Gasflusssteuerungsvorrichtung montiert werden. Insbesondere dienen die Aussparungen der Gasflussleiteinrichtung in Form von Montagenuten bzw. Montagebohrungen zur Montage und Demontage der Gasflussleiteinrichtung.

Der dritte Aspekt der Erfindung betrifft die Verwendung der zuvor beschriebenen Gasflussleiteinrichtung nach dem ersten Aspekt der Erfindung in einer Gasflusssteuerungsvorrichtung eines Beatmungsgeräts zur Beatmung eines Lebewesens.

Ein weiterer Aspekt der Erfindung betrifft die Kombination der vorhergehend beschriebenen, beispielhaft entnehmbaren, Gasleiteinheit mit mindestens einem separat entnehmbaren Gitter. Dieses mindestens eine Gitter befindet beispielsweise sich in Gasflussrichtung vor der Gasleiteinheit. Durch ein solches, herausnehmbares Gitter kann zusätzlich auch das Gitter selbst gereinigt werden und die dahinterliegenden Abschnitte ebenfalls einfach einer Reinigung zugänglich gemacht werden.

Die Gasflussleiteinrichtung kann mindestens ein (oder zwei) separat entnehmbares Gitter, welches vor dem oder innerhalb des Ringkörpers angeordnet ist aufweisen.

Das zumindest eine separat entnehmbare Gitter ist beispielsweise vor oder benachbart zu der der Leiteinheit angeordnet. Ein weiteres separat entnehmbares Gitter ist beispielsweise vor oder benachbart zu der der Leiteinheit und - bezogen auf die Leiteinheit - gegenüber dem ersten Gitter angeordnet.

Gemäß einer beispielhaften Ausführungsform ist zumindest ein separat entnehmbares Gitter in Gasflussrichtung vor der Leiteinheit und zumindest ein separat entnehmbares Gitter in Gasflussrichtung nach der Leiteinheit angeordnet. Auch die Gitter sind so einer lösbaren Montage und Demontage zugänglich und ermöglichen so eine einfache und schnelle Reinigung.

Beispielhafte Ausführungsformen der Erfindung werden beispielhaft anhand der beiliegenden Figuren beschrieben. Es zeigen:
- Figur 1: eine schematische Darstellung einer beispielhaften Ausführungsform einer Gasflusssteuerungsvorrichtung;
- Figuren 2a-2b: eine schematische Darstellung eines Ringkörpers einer Gasflussleiteinrichtung in einer Frontansicht (Fig. 2a) und einer geschnittenen Seitenansicht (Fig. 2b);
- Figuren 3a-3c: eine schematische Darstellung einer beispielhaften Ausführungsform eines Leitelements einer Leiteinheit einer Gasflussleiteinrichtung in einer Seitenansicht (Fig. 3a), einer Frontansicht (Fig. 3b) und einer Draufsicht (Fig. 3c);
- Figuren 4a-4c: eine schematische Darstellung einer weiteren beispielhaften Ausführungsform eines Leitelements einer Leiteinheit einer Gasflussleiteinrichtung in einer Seitenansicht (Fig. 4a), einer Frontansicht (Fig. 4b) und einer Draufsicht (Fig. 4c);
- Figuren 5a, 5b: eine schematische Darstellung einer beispielhaften Ausführungsform einer Gasflussleiteinrichtung in einer Frontansicht (Fig. 5a) und einer perspektivischen Ansicht (Fig. 5b);
- Figuren 6a, 6b: eine schematische Darstellung einer weiteren beispielhaften Ausführungsform einer Gasflussleiteinrichtung jeweils in einer Frontansicht;
- Figuren 7a, 7b: jeweils eine schematische, perspektivische Darstellung einer weiteren beispielhaften Ausführungsform einer Gasflussleiteinrichtung; und
- Figur 8:: eine schematische Darstellung einer weiteren beispielhaften Ausführungsform einer Gasflussleiteinrichtung in einer Frontansicht.

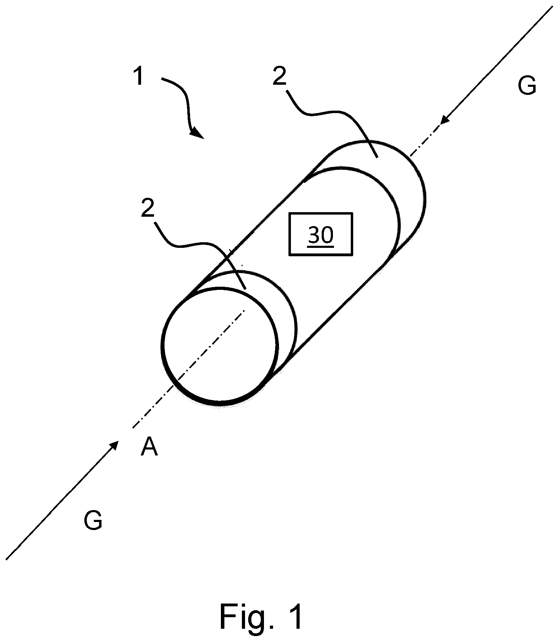

Figur 1 zeigt schematische Darstellung einer beispielhaften Ausführungsform einer Gasflussmessvorrichtung 1. Die in Figur 1 dargestellte Gasflussmessvorrichtung 1 umfasst eine Messvorrichtung 30.

Um den Volumenstrom des Gasflusses G möglichst genau zu erfassen, weist die Gasflussmessvorrichtung 1 an einem Ende eine Gasflussleiteinrichtung 2 auf. Die Gasflussleiteinrichtung 2 erzeugt einen messbaren Druckabfall, dient gleichzeitig als Strömungsberuhigung und lenkt den Gasfluss G derart, dass die Messvorrichtung 30 den Volumenstrom über den Druckabfall erfassen kann.

Die schematisch dargestellte Messvorrichtung 1 ist für einen bidirektionalen Gasfluss G ausgebildet, wie durch die beiden entgegengesetzten Pfeile angedeutet ist. Hierzu weist die Messvorrichtung 1 an beiden, jeweils gegenüberliegenden Enden jeweils eine Gasflussleiteinrichtung 2 auf, und zwar jeweils für eine Anwendung mit entgegengesetztem Gasfluss G.

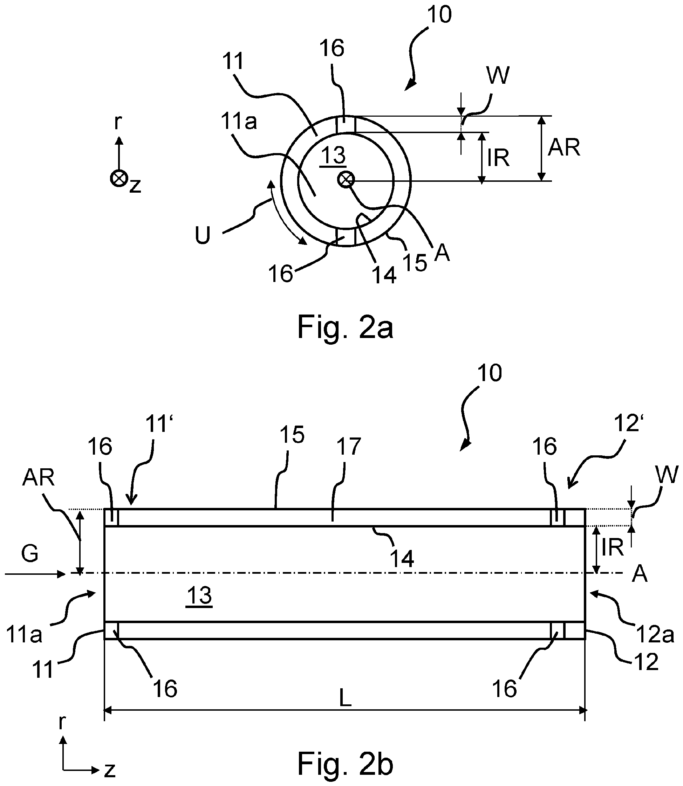

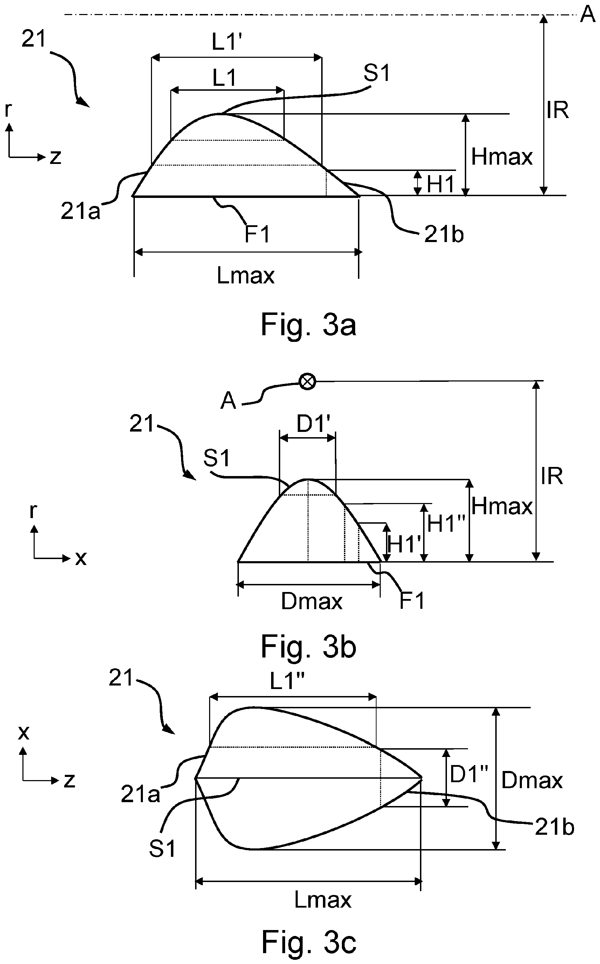

Die in Figur 1 schematisch dargestellte Gasflussleiteinrichtung 2 umfasst einen in den Figuren 2a und 2b schematisch dargestellten Ringkörper 10, sowie ferner eine in den Figuren 3a-4c zumindest teilweise schematisch dargestellte Leiteinheit 20.

Wie aus den Figuren 2a und 2b ersichtlich ist, erstreckt sich der Ringkörper 10 entlang einer Axialachse A mit einer Ringkörperlänge L. In der in den Figuren 2a und 2b schematisch dargestellten beispielhaften Ausführungsform ist der Ringkörper 10 als hohler Zylinder rohrförmig ausgebildet. Ein Außenmantel 15, der ausgehend von der Axialachse A einen Außenmantelradius AR aufweist, definiert die Außenseite des Ringkörpers 10 in radialer Richtung r entlang der Axialachse A. Ein Innenmantel 14, der ausgehend von der Axialachse A einen Innenmantelradius IR aufweist, definiert die Innenseite des Ringkörpers 10 in radialer Richtung r entlang der Axialachse A.

In den dargestellten Ausführungsbeispielen ist die radiale Richtung r jeweils in der jeweiligen Figur als Koordinatensystem eingezeichnet. Mit der Koordinate z ist regelmäßig die axiale Richtung, parallel zur Axialachse A, beschrieben.

Der in den Figuren 2a und 2b schematisch dargestellte Ringkörper 10 weist entlang der Axialachse A einen Ringkanal 13 auf. Der Ringkanal 13 erstreckt zwischen einer ersten Durchgangsöffnung 11a und einer zweiten Durchgangsöffnung 12a. Die jeweilige Durchgangsöffnung 11a, 12a bildet eine orthogonal zu der Axialachse A verlaufende Ebene aus. Alternativ könnte die durch die Durchgangsöffnung gebildete Ebene auch von einer solchen orthogonalen Lage abweichen.

Die erste und zweite Durchgangsöffnung 11a, 12a weisen jeweils einen kreisförmigen Querschnitt mit dem Innenmantelradius IR auf. Der Ringkanal 13 ist von einer zylinderförmigen Wand mit einer Wandstärke W in Umfangsrichtung U entlang der Axialachse A umschlossen. Die Wandstärke W ergibt sich in Abhängigkeit des Innenmantelradius IR und des Außenmantelradius AR, wie auch aus Figur 2a ersichtlich ist.

Der in den Figuren 2a und 2b schematisch dargestellte Ringkörper 10 erstreckt sich von einem ersten Ringkörperende 11 zu einem zweiten Ringkörperende 12.

Ausgehend von dem ersten Ringkörperende 11 weist der Ringkörper 10 einen ersten Ringkörperendabschnitt 11' auf und ausgehend von dem zweiten Ringkörperende 12 weist der Ringkörper 10 einen zweiten Ringkörperendabschnitt 12' auf. Zwischen dem ersten und zweiten Ringkörperendabschnitt 11', 12' ist ein Ringkörpermittelabschnitt 17 angeordnet. Der Ringkörpermittelabschnitt 17 ist im Wesentlichen in Umfangsrichtung U entlang der Axialachse A geschlossen.

In der in den Figuren 2a und 2b schematisch dargestellten, beispielhafte Ausführungsform des Ringkörpers 10 weist dieser an dem ersten und zweiten Ringkörperendabschnitt 11', 12' jeweils zwei gegenüberliegend angeordnete Aussparungen 16 auf. Die jeweils zwei Aussparungen sind über den Umfang des Ringkörpers 10 verteilt angeordnet und erstrecken sich von dem Außenmantel 15 bis zum Innenmantel 14. Somit bilden sie kleine Durchgangsöffnungen aus, welche dem Signalabgriff zur Übertragung des pneumatischen Signals im Bereich des jeweiligen Endabschnittes an einen nicht dargestellten Sensor dienen. Es können reine Drucksignale für die Kopplung mit Drucksensoren/Differenzdrucksensoren oder auch Flusssignale für die Kopplung mit Massenstromsensoren übertragen werden.

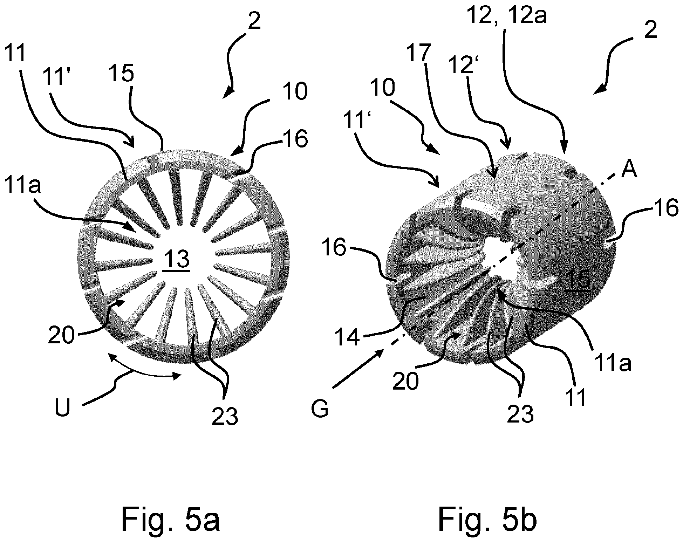

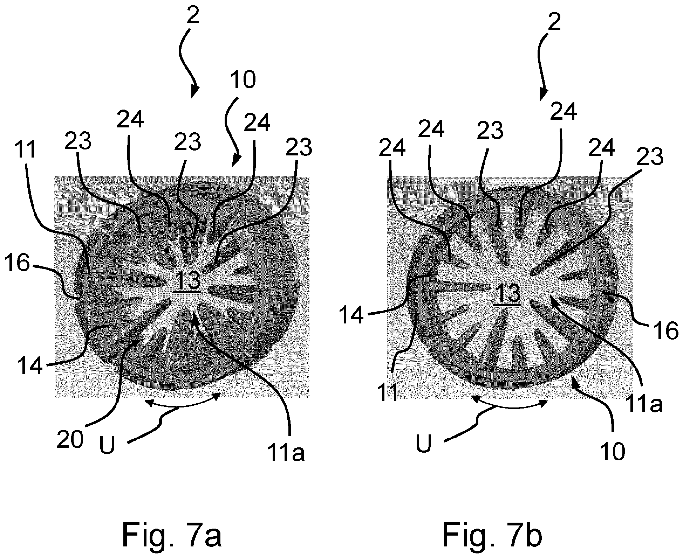

In dem ersten Ringkörperendabschnitt 11' sind die beiden Aussparungen 16 derart angeordnet, dass sie in axialer Richtung z zum ersten Ringkörperende 11 offen sind, folglich als von der in der Darstellung gemäß Figur 2b linken Stirnseite, von außen zugängliche Nuten, ausgebildet sind. In dem zweiten Ringkörperendabschnitt 12' sind die beiden Aussparungen 16 derart angeordnet, dass sie in axialer Richtung z zum zweiten Ringkörperende 12 nicht offen sind. Alternativ können jedoch auch diese Aussparungen 16 derart in dem zweiten Ringkörperendabschnitt 12' angeordnet sein, dass die Aussparungen 16 als Nuten ausgebildet sind, die zu dem zweiten Ringkörperende 12 hin in axialer Richtung z offen sind. Ein solches Ausführungsbeispiel ist im Rahmen der Figuren 5a und 5b, sowie 7a und 7b dargestellt und wird später im Detail beschrieben. Die im zweiten Ringkörperendabschnitt 12' angeordneten Aussparungen 16 sind im vorliegenden Ausführungsbeispiel der Figur 2b jedoch als Durchgangsbohrungen ausgebildet.

Bei der vorschlagsgemäßen Gasflussleiteinrichtung 2 ist innerhalb des Ringkanals 13 des Ringkörpers 10 eine Leiteinheit 20 mit mindestens einem ersten Leitelement 21 vorgesehen. Die Figuren 3a-3c zeigen eine schematische Darstellung einer ersten beispielhaften Ausführungsform eines ersten Leitelements 21 einer Leiteinheit 20 in der Seitenansicht (Fig. 3a), der Frontansicht (Fig. 3b) und der Draufsicht (Fig. 3c). Das erste Leitelement 21 erstreckt sich entlang der Axialachse A, in z-Richtung, zwischen einem ersten Leitelementende 21a und einem zweiten Leitelementende 21b mit einer ersten Leitelementlänge L1 bzw. L1' bzw. L1". Die verschiedenen Bezeichnungen L1, L1' bzw. L1" dienen dazu, zwischen den an unterschiedlichen Stellen in radialer Richtung r (vgl. Fig. 3a) bzw. in der Umfangsrichtung U bzw. in der Richtung x (vgl. Fig. 3c) verschiedenen Leitelementlängen zu unterscheiden. Die maximale Erstreckung des ersten Leitelementes 21 entlang der Axialachse A, sprich in Richtung z, liegt am ersten Leitelementfuß F1 vor und ist als Maximallänge Lmax gekennzeichnet.

Das Leitelement 21 hat in radialer Richtung r eine erste Leitelementhöhe H1 bzw. H1' bzw. H1", ausgehend von dem ersten Leitelementfuß F1 bis zu einer ersten Leitelementspitze S1. In weiteren, nicht bildlich dargestellten Ausführungsformen markiert S1 nicht eine Elementspitze im Sinne eines Scheitelpunktes, sondern bildet ein Plateau mit der Höhe Hmax und einer Länge kleiner als Lmax. In Umfangrichtung U, vgl. Fig. 2a, bzw. in der in den Fig. 3b und 3c gekennzeichneten x-Richtung weist das erste Leitelement 21 eine erste Leitelementdicke D1 bzw. D1' bzw. D1" auf. Die verschiedenen Bezeichnungen H1, H1' bzw. H1" dienen dazu, zwischen den an unterschiedlichen Stellen in Axialrichtung bzw. in der z-Richtung (vgl. Fig. 3a) bzw. in der Umfangsrichtung U bzw. der Richtung x (vgl. Fig. 3b) verschiedenen Leitelementhöhen zu unterscheiden, während die verschiedenen Bezeichnungen D1, D1' bzw. D1" dazu dienen, zwischen den an unterschiedlichen Stellen in radialer Richtung r (vgl. Fig. 3b) bzw. in Axialrichtung bzw. in der z-Richtung (vgl. Fig. 3c) verschiedenen Leitelementdicken zu unterscheiden. Die maximale Erstreckung des ersten Leitelementes 21 entlang der Umfangsrichtung U bzw. der x-Richtung ist als Maximaldicke Dmax gekennzeichnet, während die Maximalhöhe Hmax die maximale Erstreckung des ersten Leitelementes 21 in radiale Richtung r gekennzeichnet.

Eines bzw. verschiedene mehrere Leitelemente des dargestellten Typs des ersten Leitelements 21 können zwecks Ausbildung der Leiteinheit 20 mit dem ersten Leitelementfuß F1 an dem Innenmantel 14 des Ringkörpers 10 angebracht sein. Dabei können die ersten Leitelemente 21 auch einstückig mit dem Innenmantel 14 bzw. mit dem Ringkörper 10 ausgebildet sein.

Der erste Leitelementfuß F1 ist demnach zu der Axialachse A vorzugsweise mit dem Innenmantelradius IR beabstandet. Die erste Leitelementspitze S1 ist zu der Axialachse A mit der Differenz aus Innenmantelradius IR abzüglich der ersten Leitelementhöhe H1, H1', bzw. H1" beabstandet, in der Mitte des vorliegend dargestellten ersten Leitelements 21 konkret abzüglich der Maximalhöhe Hmax.

Die in den Figuren 3a-3c schematisch dargestellte, beispielhafte Ausführungsform eines ersten Leitelementes 21, welche die Leiteinheit 20 ausbildet, weist entlang der Axialachse A eine variierende erste Leitelementhöhe H1, H1', H1" auf. Ferner variiert die erste Leitelementlänge L1, L1', L1" in radialer Richtung r. Darüber hinaus kann in einer Ausführungsform die erste Leitelementdicke D1, D1', D1" entlang des Radius und/oder entlang des Axialachse A ebenfalls variieren, wobei mit zunehmendem Abstand zum Leitelementfuß bzw. entlang der Axialachse mit zunehmendem Abstand von der axialen Mitte die Dicke D1, D1', D1" beispielsweise abnimmt. Bei dem dargestellten ersten Leitelement 21 könnte es sich auch um ein zweites Leitelement handeln.

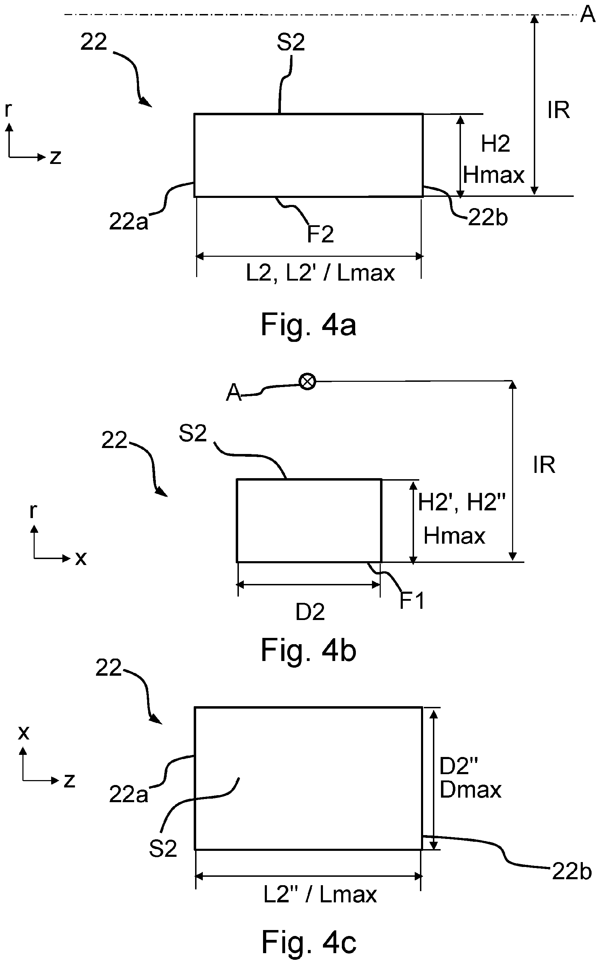

Die Figuren 4a-4c zeigen eine weitere, alternative Ausführungsform eines Leitelementes. Vorliegend ist ein Leitelement 22 einer Leiteinheit 20 in einer Seitenansicht (Fig. 4a), einer Frontansicht (Fig. 4b) und einer Draufsicht (Fig. 4c) dargestellt. Dabei verhalten sich die Lagebeziehungen und die eingezeichneten Koordinatensysteme analog zu den Darstellungen gemäß Figuren 3a-3c. Bei dem dargestellten Leitelement 22 könnte es sich auch um ein Leitelement handeln, welches entweder in Einzahl oder in Mehrzahl angebracht auf den Innenmantel 14 des Ringkörper 10 die Leiteinheit 20 der Gasflussleiteinrichtung 2 ausbilden kann. Die in den Figuren 4a-4c als scharfe Ecken und Kanten dargestellten Konturen sind in beispielhaften Ausführungsformen abgerundet und/oder rund.

Das in den Figuren 4a-4c dargestellte zweite Leitelement 22 erstreckt sich entlang der Axialachse A, in z-Richtung, zwischen einem ersten Leitelementende 22a und einem zweiten Leitelementende 22b mit einer zweiten Leitelementlänge L2 bzw. L2' bzw. L2".

Analog weist das zweite Leitelement 22 in Umfangrichtung U bzw. x-Richtung, vgl. Fig. 4a bzw. 4b, eine zweite Leitelementdicke D2 bzw. D2' bzw. D2" auf, die konstant ist und der Maximaldicke Dmax entspricht. Die zweite Leitelementhöhe H2, H2', H2" erstreckt sich von einem zweiten Leitelementfuß F2 bis zu einer zweiten Leitelementspitze S2.

Eines bzw. verschiedene mehrere Leitelemente des dargestellten Typs des zweiten Leitelements 22 können zwecks Ausbildung der Leiteinheit 20 mit dem zweiten Leitelementfuß F2 an dem Innenmantel 14 des Ringkörpers 10 angebracht sein. Dabei können die zweiten Leitelemente 22 auch einstückig mit dem Innenmantel 14 bzw. mit dem Ringkörper 10 ausgebildet sein. Weiterhin können auch eins oder mehrere Leitelemente des Typs erstes Leitelement 21 sowie auch eins oder mehrere Leitelemente des Typs zweites Leitelement 22 gleichzeitig vorgesehen sein.

Das zweite Leitelement 22 ist insbesondere mit dem zweiten Leitelementfuß F2 an dem Innenmantel 14 des Ringkörpers angeordnet. Insoweit ist der zweite Leitelementfuß F2 zu der Axialachse A mit dem Innenmantelradius IR beabstandet. Die zweite Leitelementspitze S2 ist zu der Axialachse A mit der Differenz aus Innenmantelradius IR abzüglich der zweiten Leitelementhöhe H2, H2', H2" bzw. der Maximalhöhe Hmax beabstandet.

Bezüglich der beschriebenen ersten Leitelemente 21 und der zweiten Leitelemente 22 können alternativ auch lediglich ein oder zwei Werte der ersten Leitelementhöhe H1, H1', H1" und/oder der zweiten Leitelementhöhe H2, H2', H2", der ersten Leitelementdicke D1, D1', D1" und/oder der zweiten Leitelementdicke D2, D2', D2", und/oder der ersten Leitelementlänge L1, L1', L1" und/oder der zweiten Leitelementlänge L2, L2', L2" konstant sein.

Weitere Ausführungsformen der Leitelemente (21, 22) können sich durch eine Kombination der einzelnen Seit- (Fig. 3a und 4a), Frontal- (Fig. 3b und 4b) sowie Aufsichten (Fig. 3c und 4c) ergeben. Eine beispielhafte Ausführungsform weist eine Kontur von der Seitenansicht der Figur 3a, die Frontalansicht der Figur 4b und die Aufsicht der Figur 4c auf. Eine weitere mögliche Ausführungsform kann so auch einer Kombination der Seitansicht aus Figur 4a, der Frontansicht der Figur 4b und der Aufsicht der Figur 3c folgen.

In den Figuren 5a-8 sind verschiedene beispielhafte Ausführungsformen einer Gasflussleiteinrichtung 2 dargestellt, die verschiedene beispielhafte Ausführungsformen einer Leiteinheit 20 umfassen.

Die in den Figuren 5a, 5b schematisch dargestellte beispielhafte Ausführungsform einer Gasflussleiteinrichtung 2 umfasst den Ringkörper 10, der sich entlang der Axialachse A von der ersten Ringkörperende 11 zu dem zweiten Ringkörperende 12 zylinderförmig erstreckt. In dem ersten Ringkörperendabschnitt 11' und in dem zweiten Ringkörperendabschnitt 12' sind jeweils acht Aussparungen 16 zur Weitergabe von pneumatischen Signalen (Druck und/oder Flow) in einer Gasflusssteuerungsvorrichtung 1 angeordnet, die zu dem jeweiligen ersten bzw. zweiten Ringkörperende 11 bzw. 12 hin offen sind. Die Aussparungen 16 sind somit als Nuten ausgebildet, die in axialer Richtung zu dem jeweils ersten und zweiten Ringkörperende 11 und 12 hin offen sind. Die Aussparungen 16 erstrecken sich von dem Außenmantel 15 zu dem Innenmantel 14 des Ringkörpers 10.

Der zwischen dem ersten Ringkörperendabschnitt 11' und dem zweiten Ringkörperendabschnitt 12' liegende Ringkörpermittelabschnitt 17 ist in Umfangsrichtung U entlang der Axialachse A vollständig geschlossen, d.h. der Innen- bzw. der Außenmantel 14 bzw. 15 weisen insbesondere keine Öffnung oder Durchgangsöffnungen auf. Lediglich in den Außenbereichen sind in dem ersten Ringkörperendabschnitt 11' und in dem zweiten Ringkörperendabschnitt 12' die beschriebenen Aussparungen 16 vorgesehen. Je nach Ausführung könnten sich sacklochartige Vertiefungen ergeben, um Materialanhäufungen im Spritzguss zu vermeiden; aber auch das wären keine Durchgangsöffnungen. Insbesondere bei der Herstellung im Spritzgussverfahren kann es vorteilhaft sein, dass die Breite des Leitelementfuße größer ist als die Breite der Leitelementspitze.

Die in den Figuren 5a, 5b schematisch in einer Perspektivansicht dargestellte, beispielhafte Ausführungsform einer Gasflussleiteinrichtung 2 weist eine Leiteinheit 20 in einer beispielhaften Ausführungsform mit einer Vielzahl an ersten Leitelementen 23 auf. Die Leiteinheit 20 umfasst konkret sechzehn erste Leitelemente 23. Die sechzehn ersten Leitelemente 23 sind analog zu dem zuvor im Rahmen der Figuren 3a-3c beschriebenen ersten Leitelement 21 ausgebildet. Vorliegend sind die ersten Leitelemente 23 mit ihrem jeweiligen ersten Leitelementfuß F1 an dem Innenmantel 14 äquidistant angebracht. Die sechzehn mindestens ersten Leitelemente 23 sind gleichartig ausgebildet und weisen an den entsprechenden Punkten jeweils dieselbe erste Leitelementhöhe H1, H1', bzw. H1" auf.

Durch den Ringkörper 10 wird der Ringkanal 13 ausgebildet. Die erste Durchgangsöffnung 11a und die zweite Durchgangsöffnung 12a des Ringkanals 13, durch welche im Anwendungsfall der Gasfluss G in die Gasflussleiteinrichtung 2 ein- bzw. wieder aus dieser heraustritt, bildet jeweils eine Ebene aus, die orthogonal zu der Axialachse A angeordnet ist. Der Ringkörper 10 bzw. die erste Durchgangsöffnung 11a und die zweite Durchgangsöffnung 12a weisen einen kreisförmigen Querschnitt auf.

Im Zentrum des Ringkanals 13 weist die Gasflussleiteinrichtung 2 einen Kanalabschnitt auf, in den die ersten Leitelemente 23 nicht hineinreichen bzw. hineinragen. Dieser zentrale Kanalabschnitt ist demnach vollkommen offen bzw. für eine Durchströmung des Gasflusses G frei. Die konkrete Größe dieses freien bzw. offenen Kanalabschnitts definieren die ersten Leitelementspitzen S1 der ersten Leitelemente 23. Der Kanalabschnitt weist einen Radius auf, der sich aus der Differenz des Innenmantelradius IR abzüglich der Maximalhöhe Hmax der ersten Leitelementhöhe H1, H1', H1" ergibt.

Der Ringkanal 13 weist neben dem zentralen freien bzw. offenen Kanalabschnitt ferner einen als ein Kreissegment ausgebildeten Kanalabschnitt auf, welcher keine freie Durchströmung für den Gasfluss G bereitstellt. Dieses Kreissegment ist durch die ersten Leitelemente 23 geprägt und weist in axialer Richtung gesehen eine Länge entsprechend der im vorliegenden Ausführungsbeispiel über alle ersten Leitelemente 23 betrachtet konstanten ersten Leitsegmentlänge Lmax auf und erstreckt sich ausgehend von dem Innenmantel 14 des Ringkörpers 10 in radialer Richtung mit der ersten Leitelementhöhe Hmax in Richtung der Axialachse A in den Ringkanal 13 hinein.

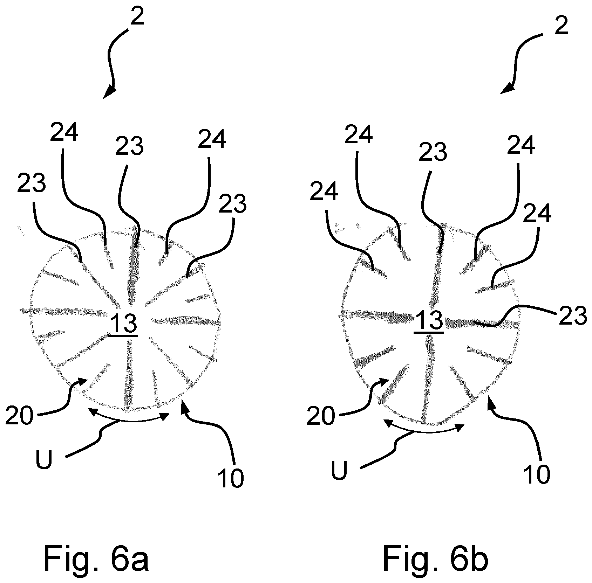

Die Figuren 6a, 6b zeigen zwei weitere schematisch jeweils in einer Frontansicht dargestellten, beispielhaften Ausführungsformen von Gasflussleiteinrichtungen 2, die in einer Gasflusssteuerungsvorrichtung 1, wie beispielsweise in Fig. 1 dargestellt, zum Einsatz kommen können. Dabei weisen die jeweiligen Ringkörper 10 der beiden Gasflussleiteinrichtungen 2 verschiedene Leiteinheiten 20 auf, wobei die jeweilige Leiteinheit 20 jeweils verschiedene Typen an Leitelementen umfasst. Konkret weist die Leiteinheit 20 der Gasflussleiteinrichtung 2 gemäß Fig. 6a acht erste Leitelemente 23 auf, die dem zuvor im Rahmen der Figuren 5a und 5b dargestellten Typ des ersten Leitelements 23 entsprechen, sowie ferner ebenfalls acht zweite Leitelemente 24. Dabei weisen die ersten Leitelemente 23 eine erste Leitelementhöhe H1, bzw. H1', bzw. H1" sowie einen entsprechenden Maximalhöhe Hmax auf, die jeweils größer sind als entsprechenden Werte der zweiten Leitelementhöhe H2, bzw. H2', bzw. H2" sowie der entsprechende Maximalhöhe Hmax der zweiten Leitelemente 24.

Bei der Ausführungsform gemäß Fig. 6b sind prinzipiell die gleichen Typen an ersten Leitelementen 23 und zweiten Leitelementen 24 vorgesehen, wie bei dem Ausführungsbeispiel gemäß Fig. 6a. Lediglich die unterschiedlichen Anzahlen sind abweichend. So sind vier erste Leitelemente 23 und 8 zweite Leitelemente 24 vorgesehen.

Bei der in Figur 6a dargestellten Ausführungsform der Leiteinheit 20 folgen in Umfangsrichtung U auf ein erstes Leitelement 23 jeweils ein zweites Leitelement 24 und auf das jeweilige zweite Leitelement 24 wiederum ein erstes Leitelement 23 und so weiter. In dieser beispielhaften Ausführungsform sind sowohl die ersten Leitelemente 23 als auch die zweiten Leitelemente 24 jeweils untereinander als auch bezüglich der verschiedenen Typen an Leitelementen zueinander äquidistant angeordnet.

Im Ausführungsbeispiel gemäß Figur 6b sind zwischen zwei ersten Leitelementen 23 jeweils zwei zweite Leitelemente 24 angeordnet. Auch hier sind die ersten Leitelemente 23 in Umfangsrichtung U gesehen jeweils zueinander äquidistant angeordnet, während die zweiten Leitelemente 24 zueinander abweichende Abstände haben können. Die Gesamtheit aller ersten Leitelemente 23 und zweiten Leitelemente 24 ist jedoch erneut in beispielhafter Weise in Umfangsrichtung U in äquidistanten Abständen untereinander entlang des Innenmantels 14 angeordnet.

Figuren 7a und 7b zeigen perspektivische Ansichten von Ausführungsbeispielen an Gasflussleiteinrichtungen 2. Dabei ist in Figur 7a die Gasflusseinrichtung 2 gemäß zuvor diskutierter Figur 6a dargestellt. In Figur 7b wiederum ist ein der in Figur 6b dargestellten Gasflusseinrichtung 2 ähnliches Ausführungsbeispiel gezeigt, wobei in der beispielhaften Ausführungsform gemäß Figur 7b die Leiteinheit 20 nun fünf, in Umfangrichtung U äquidistant verteilte, erste Leitelemente 23 aufweist, und zehn zweite Leitelemente 24 umfasst. Dabei sind erneut jeweils zwei zweite Leitelemente 24 zwischen zwei ersten Leitelementen 23 angeordnet. Die Gesamtheit aller ersten Leitelemente 23 und zweiten Leitelemente 24 ist erneut in beispielhafter Weise in Umfangsrichtung U in äquidistanten Abständen untereinander auf dem Innenmantel 14 angebracht.

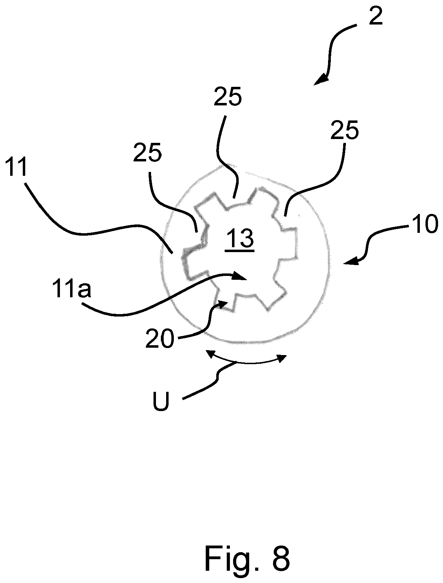

Die in Figur 8 schematisch dargestellte Ausführungsform einer Gasflussleiteinrichtung 2 umfasst eine Leiteinheit 20, die beispielhaft sechs Leitelemente 25 aufweist. Diese Leitelemente 25 weisen entlang der Axialachse A und in radialer Richtung r eine optional konstante zweite Leitelementdicke D2, bzw. D2', bzw. D2" und entsprechend optional einen konstanten Maximalbreite Dmax auf. Die Länge und Höhe des Leitelements ist in radialer Richtung variabel. Die Leitelemente 25 sind bespielhaft von dem Typ einer Kombination der in Figur 3a dargestellten Seitansicht mit der in Figuren 4b und 4c gezeigten Frontal- und Aufsicht. In beispielhafter Weise wird solch eine Leiteinheit 20 zerspanend erzeugt, insbesondere durch zerspanendes Einbringen von Nuten entlang der Axialachse A auf dem Innenmantel 14 des Ringkörpers 10. Alternativ kann auch sein, eine solche Ausführungsform der Leiteinheit 20 spritzgießend herzustellen. Wiederum alternativ sind auch Rapid Manufacturing Verfahren denkbar und können vorteilhaft sein.

Im Allgemeinen könnten bei den zuvor diskutierten Ausführungsbeispielen an Gasflussleiteinrichtungen 2 gemäß der Figuren 5a-7b die jeweils dargestellten ersten Leitelemente 23 bzw. zweiten Leitelemente 24 des Typs eines Leitelements sein, wie im Rahmen des Leitelementes 21 in den Figuren 3a-3c dargestellt und diskutiert, sprich mit variablen Leitelementlängen, -höhen und optional Leitelementdicken, oder aber auch des Typs eines Leitelements sein, wie im Rahmen des zweiten Leitelementes 22 in den Figuren 4a-4c dargestellt und diskutiert, oder aber auch einer Mischung aus den verschiedenen Typen an Leitelementen entsprechen. Wie vorangehend beschrieben sind die Ausführungen der Leitelemente nicht auf die durch Abbildungen 3a-3c sowie 4a-4c diskutierten Beispiele beschränkt, sondern können sich auch durch Kombinationen der jeweiligen Konturen bzw. Profile ergeben. Bei Einsatz mehrerer gleicher Leitelemente können diese untereinander in ihren Leitelementlängen, -höhen und -dicken variieren.

Weiterhin kann auch die Anzahl der der Leitelemente variieren. Eine beispielhafte, aber nicht darauf limitierte, gesamte Anzahl von Leitelementen ist 4-32 Leitelemente. Bevorzugt sind 8 bis 24 Leitelemente, weiter bevorzugt sind 12 bis 18 Leitelemente.

Die beschriebenen Gasflussleiteinrichtungen 2 können als austauschbare Einheiten in Gasflusssteuerungsvorrichtungen 1 vorgesehen werden und somit in Beatmungsgeräten zur Beatmung von Lebewesen zum Einsatz kommen. Der Vorteil der Gasflussleiteinrichtungen 2 ist, dass diese austauschbaren Einheiten leichter zu montieren und demontieren und vor allem zu reinigen bzw. zu desinfizieren sind. Außerdem kann in vorteilhafter Weise auf den Einsatz von den gesamten Strömungsquerschnitt einnehmenden, sogenannten Beruhigungsgittern, verzichtet werden. Alternativ können die beschriebenen Gasflusseinrichtungen mit austauschbaren Gittern, kombiniert werden, sodass auch jene Gitter einer einfachen Reinigung und Desinfizierung zugänglich sind. Beispielsweise können dabei zwei und mehr solcher Gitter verwendet werden, wobei diese zum Teil vor und zum Teil nach der Leiteinheit 20 angeordnet sind und in vorteilhafter Weise separat entnehmbar sind. In vorteilhafter Weise ist bei den Leiteinheiten 20 der Gasflussleiteinrichtungen 2 ein zentraler, offener Bereich bzw. Kanalabschnitt vorgesehen, wodurch sich im Vergleich zu sich über den gesamten Querschnitt erstreckenden Gittern Schmutz nicht so leicht ansammeln und den Strömungsquerschnitt verkleinern oder gar verstopfen kann. Durch die vorschlagsgemäßen Gasflussleiteinrichtungen 2 ist somit die Gefahr an Verstopfungen im Einsatz deutlich verringert, wodurch die sogenannte Flowmessstrecke des Beatmungsgerätes ein besseres, unverfälschtes Signal für den Gasfluss G erzeugt, es entstehen damit seltener Fehlsignale. Dadurch, dass die Gasflussleiteinrichtungen 2 beispielsweise austauschbar, sprich auch wieder aus einer Gasflussteuerungsvorrichtung entnehmbar und erneut einsetzbar, sind, müssen die Komponenten im Unterschied zu verstopften Beruhigungsgittern des Standes der Technik nicht mehr entsorgt und durch neue ersetzt werden, sondern können vielmehr gereinigt und wiederverwendet werden. Werden die Gasflussleiteinrichtungen mit mindestens einem in Gasflussrichtung davorliegenden austauschbaren Gitter kombiniert verwendet, so kann eine Reinigung der Vorrichtung auch ohne Ausbau möglich sein. Beispielhaft können auch zwei austauschbare, gegebenenfalls separat entnehmbare Gitter mit den Gasflussleiteinrichtungen kombiniert bzw. in diese integriert werden. Eines der Gitter kann dabei in Gasflussrichtung vor und eines der Gitter in Gasflussrichtung nach der Leiteinheit/Gasflussleiteinrichtung angeordnet sein. Der zentrale, freie Kanalabschnitt, welche durch die Leiteinheiten 20 nicht belegt ist, erleichtert auch die Reinigung der entsprechenden Gasflussleiteinrichtungen 2.

## Patentansprüche

1. Gasflussleiteinrichtung (2) zum Leiten eines Gasflusses (G) in einem Therapiegerät zur Atemgaszufuhr bei Lebewesen, aufweisend einen Ringkörper (10) und eine innerhalb des Ringkörpers (10) angeordnete Leiteinheit (20) zum Leiten des durch den Ringkörper (10) geführten Gasflusses (G), wobei die Leiteinheit (20) mindestens ein erstes Leitelement (21, 23) und mindestens ein zweites Leitelement (22,24) umfasst, wobei
- das mindestens eine erste Leitelement (21,23) sich in einer radialen Richtung (r) mit einer ersten Leitelementhöhe (H1, H1', H1") von einem ersten Leitelementfuß (F1) zu einer ersten Leitelementspitze (S1) erstreckt, und eine Maximalhöhe (Hmax) der ersten Leitelementhöhe (H1, H1', H1") aufweist;
- das mindestens ein zweites Leitelement (22,24) sich in der radialen Richtung (r) mit einer zweiten Leitelementhöhe (H2, H2', H2") von einem zweiten Leitelementfuß (F2) zu einer mindestens zweiten Leitelementspitze (S2) erstreckt, und eine Maximalhöhe (Hmax) der zweiten Leitelementhöhe (H2, H2', H2") aufweist;
**dadurch gekennzeichnet, dass**
die Maximalhöhe (Hmax) der ersten Leitelementhöhe (H1, H1', H1") des mindestens einen ersten Leitelements (21, 23) größer ist als die Maximalhöhe (Hmax) der zweiten Leitelementhöhe (H2, H2', H2") des mindestens einen zweiten Leitelements (22,24).

2. Gasflussleiteinrichtung (2) nach Anspruch 1, wobei:
- das mindestens ein erstes Leitelement sich entlang der Axialachse (A) mit einer ersten Leitelementlänge (L1, L1', L1") von einem ersten Leitelementende zu einem zweiten Leitelementende erstreckt, und
- der Innenmantel (14) ausgehend von der Axialachse (A) einen Innenmantelradius (IR) aufweist, der größer ist als die Maximalhöhe (Hmax) der ersten Leitelementhöhe (H1, H1', H1") des mindestens einen ersten Leitelements (21,23).

3. Gasflussleiteinrichtung (2) nach Anspruch 2, aufweisend:
- einen Ringkörper (10), der zur Führung des Gasflusses (G) ausgebildet ist und der sich entlang einer Axialachse (A) mit einer Ringkörperlänge (L) von einem ersten Ringkörperende (11) zu einem zweiten Ringkörperende (12) erstreckt, wobei der Ringkörper (10) an dem ersten Ringkörperende (11) eine erste Durchgangsöffnung (IIa) und an dem zweiten Ringkörperende (12) eine zweite Durchgangsöffnung (12a) aufweist, zwischen denen sich ein innerhalb des Ringkörpers (10) angeordneter Ringkanal (13) erstreckt, wobei der Innenmantel (14) des Ringköpers (10) den Ringkanal (13) ausgehend von der Axialachse (A) in einer radialen Richtung (r) entlang der Axialachse (A) begrenzt, und
- die Leiteinheit (20) innerhalb des Ringkanals (13) angeordnet ist, und das mindestens eine Leitelement (21,23) in Umfangsrichtung (U) eine erste Leitelementdicke (D1, D1', D1") aufweist.

4. Gasflussleiteinrichtung (2) nach mindestens einem der Ansprüche 1 bis 3, die eingerichtet und ausgebildet ist einen flussabhängigen Druckabfall zu generieren, wobei der Ringkörper einen kreisförmigen Querschnitt oder einen polygonalen Querschnitt oder einen rechteckigen Querschnitt oder einen elliptischen Querschnitt besitzt.

5. Gasflussleiteinrichtung (2) nach Anspruch 3 oder nach den Ansprüchen 3 und 4, wobei das mindestens eine zweite Leitelement (22, 24) sich entlang der Axialachse (A) mit einer zweiten Leitelementlänge (L2, L2', L2") von einem ersten Leitelementende (22a) zu einem zweiten Leitelementende (22b) erstreckt, und in Umfangsrichtung (U) eine zweite Leitelementdicke (D2, D2', D2") aufweist, wobei der Innenmantelradius (IR) des Innenmantels (14) größer ist als die Maximalhöhe (Hmax) der zweiten Leitelementhöhe (H2, H2', H2") des mindestens einen zweiten Leitelements (22,24).

6. Gasflussleiteinrichtung (2) nach mindestens einem der vorhergehenden Ansprüche 1 bis 6, wobei das mindestens eine erste Leitelement (21, 23) mit dem ersten Leitelementfuß (F1) und/oder das mindestens eine zweite Leitelement (22, 24) mit dem zweiten Leitelementfuß (F2) an dem Innenmantel (14) des Ringkanals (13) angebracht ist und sich radial in den Ringkanal, insbesondere radial in Richtung der Axialachse (A), erstreckt.

7. Gasflussleiteinrichtung (2) nach Anspruch 2, oder Anspruch 3, oder einem der Ansprüche 4 bis 6 wenn von Anspruch 2 oder 3 abhängig, wobei die Maximalhöhe (Hmax) der ersten Leitelementhöhe (H1, H1', H1") des mindestens einen ersten Leitelements (23,25) mindestens 50 %, vorzugsweise mindestens 75%, des Innenmantelradius (IR) beträgt und/oder die Maximalhöhe (Hmax) der zweiten Leitelementhöhe (H2, H2', H2") des mindestens einen zweiten Leitelements (22, 24) zwischen 15 % und 75 %, vorzugsweise zwischen 25 % und 50%, des Innenmantelradius (IR) beträgt.

8. Gasflusseinrichtung (2) nach mindestens einem der vorhergehenden Ansprüche, wobei der Ringkörper (10) als hohler Zylinder rohrförmig und als Rohr ausgebildet ist und der Innendurchmesser des Rohres mit kreisförmigem Querschnitt zwischen 10 und 34 mm, bevorzugt 16 bis 24 mm beträgt.

9. Gasflusseinrichtung (2) nach mindestens einem der vorhergehenden Ansprüche, wobei die Leitelemente eine maximale Wandstärke (Dmax) von 0,4 mm bis 2,5 mm, bevorzugt von 0,8 bis 1,8 mm aufweisen und wobei die Leitelemente eine Länge (Lmax) im Bereich des Leitelementfußes (F1,F2) von 4 bis 50 mm, bevorzugt 12 bis 30 mm aufweisen.

10. Gasflusseinrichtung (2) nach mindestens einem der vorhergehenden Ansprüche, wobei die Gasleiteinheit mit 4 bis 32, bevorzugt 8 bis 24, noch mehr bevorzugt 12 bis 18 Leitelementen ausgestattet ist, wobei die Leitelemente (21, 22, 23, 24) äquidistant über den Umfang angeordnet sind.

11. Gasflussleiteinrichtung (2) nach mindestens einem der vorhergehenden Ansprüche, wobei die Leiteinheit (20) eine erste Anzahl an ersten Leitelementen (21,23) und eine zweite Anzahl an zweiten Leitelementen (22,24) aufweist, wobei die erste Anzahl an ersten Leitelementen (21,23) und die zweite Anzahl an zweiten Leitelementen (22,24) gleich sind oder die zweite Anzahl an zweiten Leitelementen (22,24) größer ist als die erste Anzahl an ersten Leitelementen (21,23).

12. Gasflussleiteinrichtung (2) nach mindestens einem der vorhergehenden Ansprüche, wobei zwischen zwei in Umfangsrichtung (U) benachbart angeordneten, ersten Leitelementen (21,23) ein zweites Leitelement (22,24), oder zwei zweite Leitelemente (22,24), oder mehr als zwei zweite Leitelemente (22,24) angeordnet sind.

13. Gasflussleiteinrichtung (2) nach mindestens einem der vorhergehenden Ansprüche, wobei
- die erste Leitelementlänge (L1, L1', L1") des mindestens einen ersten Leitelements (21, 23) in radialer Richtung (r) entlang der ersten Leitelementhöhe (H1, H1', H1") variiert oder konstant ist und/oder die zweite Leitelementlänge (L2, L2', L2") des mindestens einen zweiten Leitelements (22, 24) in radialer Richtung (r) entlang der zweiten Leitelementhöhe (H2, H2', H2") variiert oder konstant ist; und/oder
- die erste Leitelementhöhe (H1, H 1', H1") des mindestens einen ersten Leitelements (21, 23) und/oder die zweite Leitelementhöhe (H2, H2', H2") des mindestens einen zweiten Leitelements (22, 24) entlang der Axialachse (A) variiert oder konstant ist; und/oder
- die erste Leitelementdicke (D1, D1', D1") des mindestens einen ersten Leitelements (21, 23) in radialer Richtung (r) entlang der ersten Leitelementhöhe (H1, H1', H1") variiert oder konstant ist und/oder die zweite Leitelementdicke (D2, D2', D2") des mindestens einen zweiten Leitelements (22, 24) in radialer Richtung (r) entlang der ersten Leitelementhöhe (H2, H2', H2") variiert oder konstant ist.

14. Gasflussleiteinrichtung (2) nach mindestens einem der vorhergehenden Ansprüche, wobei die Leiteinheit (20) einstückig mit dem Ringkörper (10) ausgebildet ist oder in den Ringkanal (13) des Ringkörpers (10) eingesetzt ist, wobei die einstückig mit dem Ringkörper (10) ausgebildete Leiteinheit (20) spanend und/oder gießend und/oder fügend erzeugt ist.

15. Gasflussleiteinrichtung (2) nach mindestens einem der vorhergehenden Ansprüche, wobei der Ringkörper (10) einen Außenmantel (15) mit einem Außenmantelradius (AR) aufweist, wobei der Außenmantel (15) vorzugsweise zylinderförmig ausgebildet ist.

16. Gasflussleiteinrichtung (2) nach mindestens Anspruch 3 und Anspruch 15 wobei der Ringkörper (10) ausgehend von dem ersten Ringkörperende (11) einen ersten Ringkörperendabschnitt (11') und ausgehend von dem zweiten Ringkörperende (12) einen zweiten Ringkörperendabschnitt (12') aufweist, zwischen denen sich ein Ringkörpermittelabschnitt (17) erstreckt, wobei der erste Ringkörperendabschnitt (11') und/oder zweite Ringkörperendabschnitt (12') über den Umfang verteilt wenigstens eine sich von dem Außenmantel (15) bis zu dem Innenmantel (14) des Ringkörpers (10) erstreckende Aussparung oder Bohrung (16) aufweist.

17. Gasflussleiteinrichtung (2) nach mindestens einem der vorhergehenden Ansprüche, aufweisend mindestens ein separat entnehmbares Gitter, welches vor oder innerhalb des Ringkörpers (10) angeordnet ist.

18. Gasflussleiteinrichtung (2) nach Anspruch 17, wobei zumindest ein separat entnehmbares Gitter in Gasflussrichtung vor der Leiteinheit (20) angeordnet ist und zumindest ein separat entnehmbares Gitter in Gasflussrichtung nach der Leiteinheit (20) angeordnet ist.

## Claims

1. A gas flow guiding device (2) for guiding a gas flow (G) in a therapy device for supplying respiratory gas for living organisms, comprising an annular body (10) and a guiding unit (20) arranged inside the annular body (10) for guiding the gas flow (G) conveyed through the annular body (10), wherein the guiding unit (20) comprises at least one first guiding element (21, 23) and at least one second guiding element (22, 24), wherein
- the at least one first guiding element (21, 23) extends in a radial direction (r) with a first guiding element height (H1, H1', H1") from a first guiding element foot (F1) to a first guiding element tip (S1) and has a maximum height (Hmax) of the first guiding element height (H1, H1', H1");
- the at least one second guiding element (22, 24) extends in the radial direction (r) with a second guiding element height (H2, H2', H2") from a second guiding element foot (F2) to an at least second guiding element tip (S2) and has a maximal height (Hmax) of the second guiding element height (H2, H2', H2");
**characterized in that**
the maximal height (Hmax) of the first guiding element height (H1, H1', H1") of the at least one first guiding element (21, 23) is larger than the maximal height (Hmax) of the second guiding element height (H2, H2', H2") of the at least one second guiding element (22, 24).

2. The gas flow guiding device (2) according to claim 1, wherein:
- the at least one first guiding element extends along the axial axis (A) with a first guiding element length (L1, L1', L1") of a first guiding element end to a second guiding element end, and
- the inner shell (14) has an inner shell radius (IR) proceeding from the axial axis (A), which inner shell radius is larger than the maximal height (Hmax) of the first guiding element height (H1, H1', H1") of the at least one first guiding element (21, 23).

3. The gas flow guiding device (2) according to claim 2, having:
- an annular body (10) which is designed to convey the gas flow (G) and which extends along an axial axis (A) with an annular body length (L) from a first annular body end (11) to a second annular body end (12), wherein the annular body (10) has a first through-opening (11a) on the first annular body end (11) and a second through-opening (12a) on the second annular body end (12), between which through-openings an annular channel (13) extends which is arranged inside the annular body (10), wherein the inner shell (14) of the annular body (10) delimits the annular channel (13) proceeding from the axial axis (A) along the axial axis (A) in a radial direction (r), and
- the guiding unit (20) is arranged inside the annular channel (13) and the at least one guiding element (21, 23) has a first guiding element thickness (D1, D1', D1") in the circumferential direction (U).

4. The gas flow guiding device (2) according to at least one of claims 1 to 3, which is configured and designed to generate a flow-dependent pressure drop, wherein the annular body has a circular cross-section or a polygonal cross-section or a rectangular cross-section or an elliptical cross-section.

5. The gas flow guiding device (2) according to claim 3 or according to claims 3 and 4, wherein the at least one second guiding element (22, 24) extends along the axial axis (A) with a second guiding element length (L2, L2', L2") from a first guiding element end (22a) to a second guiding element end (22b) and has a second guiding element thickness (D2, D2', D2") in the circumferential direction (U), wherein the inner shell radius (IR) of the inner shell (14) is larger than the maximum height (Hmax) of the second guiding element height (H2, H2', H2") of the at least one second guiding element (22, 24).

6. The gas flow guiding device (2) according to at least one of the preceding claims 1 to 6, wherein the at least one first guiding element (21, 23) is attached with the first guiding element foot (F1) and/or the at least one second guiding element (22, 24) is attached with the second guiding element foot (F2) to the inner shell (14) of the annular channel (13) and extends radially in the annular channel, in particular radially in the direction of the axial axis (A).

7. The gas flow guiding device (2) according to claim 2 or claim 3, or one of claims 4 to 6 if dependent on claim 2 or 3, wherein the maximum height (Hmax) of the first guiding element height (H1, H1', H1") of the at least one first guiding element (23, 25) is at least 50%, preferably at least 75% of the inner shell radius (IR), and/or the maximum height (Hmax) of the second guiding element height (H2, H2', H2") of the at least one second guiding element (22, 24) is between 15% and 75%, preferably between 25% and 50% of the inner shell radius (IR).

8. The gas flow guiding device (2) according to at least one of the preceding claims, wherein the annual body (10) is designed as a hollow cylinder in a tubular manner and as a tube and the inner diameter of the tube with a circular cross-section is between 10 and 34 mm, preferably between 16 and 24 mm.

9. The gas flow guiding device (2) according to at least one of the preceding claims, wherein the guiding elements have a maximum wall thickness (Dmax) of 0.4 mm to 2.5 mm, preferably 0.8 mm to 1.8 mm, and wherein the guiding elements have a length (Lmax) in the region of the guiding element foot (F1, F2) of 4 to 50 mm, preferably 12 to 30 mm.

10. The gas flow guiding device (2) according to at least one of the preceding claims, wherein the gas flow unit is equipped with 4 to 32, preferably 8 to 24, more preferably 12 to 18 guiding elements, wherein the guiding elements (21, 22, 23, 24) are arranged so as to be equidistant to one another over the circumference.

11. The gas flow guiding device (2) according to at least one of the preceding claims, wherein the guiding unit (20) has a first number of first guiding elements (21, 23) and a second number of second guiding elements (22, 24), wherein the first number of first guiding elements (21, 23) and the second number of second guiding elements (22, 24) is the same or the second number of second guiding elements (22, 24) is larger than the first number of first guiding elements (21,23).

12. The gas flow guiding device (2) according to at least one of the preceding claims, wherein a second guiding element (22, 24) or two second guiding elements (22, 24) or more than two second guiding elements (22, 24) are arranged between two first guiding elements (21, 23) arranged adjacent to each other in the circumferential direction (U).

13. The gas flow guiding device (2) according to at least one of the preceding claims, wherein
- the first guiding element length (L1, L1', L1") of the at least one first guiding element (21, 23) varies or is constant in the radial direction (r) along the first guiding element height (H1, H1', H1"), and/or the second guiding element length (L2, L2', L2") of the at least one second guiding element (22, 24) varies or is constant in the radial direction (r) along the second guiding element height (H2, H2', H2"); and/or
- the first guiding element height (H1, H1', H1") of the at least one first guiding element (21, 23) and/or the second guiding element height (H2, H2', H2") of the at least one second guiding element (22, 24) varies or is constant along the axial axis (A); and/or
- the first guiding element thickness (D1, D1', D1") of the at least one first guiding element (21, 23) varies or is constant in the radial direction (r) along the first guiding element height (H1, H1', H1"), and/or the second guiding element thickness (D2, D2', D2") of the at least one second guiding element (22, 24) varies or is constant in the radial direction (r) along the first guiding element height (H2, H2', H2").

14. The gas flow guiding device (2) according to at least one of the preceding claims, wherein the guiding unit (20) is formed integrally with the annular body (10) or is inserted into the annular channel (13) of the annular body (10), wherein the guiding unit (20) that is formed integrally with the annular body (10) is produced by machining and/or casting and/or joining.

15. The gas flow guiding device (2) according to at least one of the preceding claims, wherein the annular body (10) has an outer shell (15) with an outer shell radius (AR), wherein the outer shell (15) is preferably designed to be cylindrical.

16. The gas flow guiding device (2) according to at least claim 3 and claim 15, wherein the annular body (10) has a first annular body end section (11') proceeding from the first annular body end (11) and a second annular body end section (12') proceeding from the second annular body end (12), between which end sections an annular body middle section (17) extends, wherein the first annular body end section (11') and/or second annular body end section (12') comprises, distributed over the circumference, at least one recess or hole (16) extending from the outer shell (15) to the inner shell (14) of the annular body (10).

17. The gas flow guiding device (2) according to at least one of the preceding claims, comprising at least one separately removable grating which is arranged in front of or inside the annular body (10).

18. The gas flow guiding device (2) according to claim 17, wherein at least one separately removable grating is arranged in front of the guiding unit (20) in the gas flow direction and at least one separately removable grating is arranged behind the guiding unit (20) in the gas flow direction.

## Revendications

1. Dispositif de guidage de flux de gaz (2) destiné au guidage d'un flux de gaz (G) dans un appareil thérapeutique pour l'alimentation en gaz respiratoire chez des êtres vivants, présentant un corps annulaire (10) et une unité de guidage (20) disposée à l'intérieur du corps annulaire (10) pour le guidage du flux de gaz (G) dirigé à travers le corps annulaire (10), dans lequel l'unité de guidage (20) comporte au moins un premier élément de guidage (21, 23) et au moins un deuxième élément de guidage (22, 24), dans lequel
- l'au moins un premier élément de guidage (21, 23) s'étend dans une direction radiale (r) avec une première hauteur d'élément de guidage (H1, H1', H1") à partir d'une première base d'élément de guidage (F1) vers une première pointe d'élément de guidage (S1), et présente une hauteur maximale (Hmax) de la première hauteur d'élément de guidage (H1, H1', H1") ;
- l'au moins un deuxième élément de guidage (22, 24) s'étend dans la direction radiale (r) avec une deuxième hauteur d'élément de guidage (H2, H2', H2") à partir d'une deuxième base d'élément de guidage (F2) vers une au moins deuxième pointe d'élément de guidage (S2), et présente une hauteur maximale (Hmax) de la deuxième hauteur d'élément de guidage (H2, H2', H2") ;
**caractérisé en ce que**
la hauteur maximale (Hmax) de la première hauteur d'élément de guidage (H1, H1', H1") de l'au moins un premier élément de guidage (21, 23) est supérieure à la hauteur maximale (Hmax) de la deuxième hauteur d'élément de guidage (H2, H2', H2") de l'au moins un deuxième élément de guidage (22, 24).

2. Dispositif de guidage de flux de gaz (2) selon la revendication 1, dans lequel :
- l'au moins un premier élément de guidage s'étend le long de l'axe axial (A) avec une première longueur d'élément de guidage (L1, L1', L1") à partir d'une première extrémité d'élément de guidage jusqu'à une deuxième extrémité d'élément de guidage, et
- l'enveloppe intérieure (14) à partir de l'axe axial (A) présente un rayon d'enveloppe intérieure (IR) supérieur à la hauteur maximale (Hmax) de la première hauteur d'élément de guidage (H1, H1', H1") de l'au moins un premier élément de guidage (21, 23).

3. Dispositif de guidage de flux de gaz (2) selon la revendication 2, présentant :
- un corps annulaire (10) conçu pour diriger le flux de gaz (G) et s'étendant le long d'un axe axial (A) avec une longueur de corps annulaire (L) à partir d'une première extrémité de corps annulaire (11) vers une deuxième extrémité de corps annulaire (12), dans lequel le corps annulaire (10) présente une première ouverture de passage (11a) à la première extrémité de corps annulaire (11) et une deuxième ouverture de passage (12a) à la deuxième extrémité de corps annulaire (12), entre lesquelles s'étend un canal annulaire (13) disposé à l'intérieur du corps annulaire (10), dans lequel l'enveloppe intérieure (14) du corps annulaire (10) délimite le canal annulaire (13) à partir de l'axe axial (A) dans une direction radiale (r) le long de l'axe axial (A), et
- l'unité de guidage (20) est disposée à l'intérieur du canal annulaire (13), et l'au moins un élément de guidage (21, 23) présente une première épaisseur d'élément de guidage (D1, D1', D1") dans la direction circonférentielle (U).

4. Dispositif de guidage de flux de gaz (2) selon l'une au moins des revendications 1 à 3, lequel est configuré et conçu pour générer une baisse de pression en fonction du flux, dans lequel le corps annulaire possède une section transversale circulaire ou une section transversale polygonale ou une section transversale rectangulaire ou une section transversale elliptique.

5. Dispositif de guidage de flux de gaz (2) selon la revendication 3 ou selon les revendications 3 et 4, dans lequel l'au moins un deuxième élément de guidage (22, 24) s'étend le long de l'axe axial (A) avec une deuxième longueur d'élément de guidage (L2, L2', L2") à partir d'une première extrémité d'élément de guidage (22a) vers une deuxième extrémité d'élément de guidage (22b), et présente une deuxième épaisseur d'élément de guidage (D2, D2', D2") dans la direction circonférentielle (U), dans lequel le rayon d'enveloppe intérieure (IR) de l'enveloppe intérieure (14) est supérieur à la hauteur maximale (Hmax) de la deuxième hauteur d'élément de guidage (H2, H2', H2") de l'au moins un deuxième élément de guidage (22, 24).

6. Dispositif de guidage de flux de gaz (2) selon l'une au moins des revendications précédentes 1 à 6, dans lequel l'au moins un premier élément de guidage (21, 23) est fixé avec la première base d'élément de guidage (F1) et/ou l'au moins un deuxième élément de guidage (22, 24) est fixé avec la deuxième base d'élément de guidage (F2) à l'enveloppe intérieure (14) du canal annulaire (13) et s'étend radialement dans le canal annulaire, en particulier radialement en direction de l'axe axial (A).

7. Dispositif de guidage de flux de gaz (2) selon la revendication 2, ou la revendication 3, ou l'une des revendications 4 à 6 dans la mesure où celle-ci dépend de la revendication 2 ou 3, dans lequel la hauteur maximale (Hmax) de la première hauteur d'élément de guidage (H1, H1', H1") de l'au moins un premier élément de guidage (21, 23) mesure au moins 50%, de préférence au moins 75%, du rayon d'enveloppe intérieure (IR) et/ou la hauteur maximale (Hmax) de la deuxième hauteur d'élément de guidage (H2, H2', H2") de l'au moins un deuxième élément de guidage (22, 24) mesure entre 15% et 75%, de préférence entre 25% et 50%, du rayon d'enveloppe intérieure (IR).

8. Dispositif de guidage de flux de gaz (2) selon l'une au moins des revendications précédentes, dans lequel le corps annulaire (10) est conçu tubulaire comme un cylindre creux et comme un tuyau et le diamètre intérieur du tuyau à section transversale circulaire mesure entre 10 et 34 mm, de préférence de 16 à 24 mm.

9. Dispositif de guidage de flux de gaz (2) selon l'une au moins des revendications précédentes, dans lequel les éléments de guidage présentent une épaisseur de paroi maximale (Dmax) de 0,4 mm à 2,5 mm, de préférence de 0,8 à 1,8 mm et dans lequel les éléments de guidage présentent une longueur (Lmax) de 4 à 50 mm, de préférence de 12 à 30 mm, dans la région de la base d'élément de guidage (F1, F2).

10. Dispositif de guidage de flux de gaz (2) selon l'une au moins des revendications précédentes, dans lequel l'unité de guidage de gaz est équipée de 4 à 32, de préférence de 8 à 24, plus préférentiellement de 12 à 18 éléments de guidage, dans lequel les éléments de guidage (21, 22, 23, 24) sont disposés de manière équidistante sur la circonférence.

11. Dispositif de guidage de flux de gaz (2) selon l'une au moins des revendications précédentes, dans lequel l'unité de guidage (20) présente un premier nombre de premiers éléments de guidage (21, 23) et un deuxième nombre de deuxièmes éléments de guidage (22, 24), dans lequel le premier nombre de premiers éléments de guidage (21, 23) et le deuxième nombre de deuxièmes éléments de guidage (22, 24) sont identiques ou le deuxième nombre de deuxièmes éléments de guidage (22, 24) est supérieur au premier nombre de premiers éléments de guidage (21, 23).

12. Dispositif de guidage de flux de gaz (2) selon l'une au moins des revendications précédentes, dans lequel un deuxième élément de guidage (22, 24), ou deux deuxièmes éléments de guidage (22, 24), ou plus de deux deuxièmes éléments de guidage (22, 24) sont disposés entre deux premiers éléments de guidage (21, 23) disposés de façon adjacente dans la direction circonférentielle (U).

13. Dispositif de guidage de flux de gaz (2) selon l'une au moins des revendications précédentes, dans lequel
- la première longueur d'élément de guidage (L1, L1', L1") de l'au moins un premier élément de guidage (21, 23) varie ou est constante dans la direction radiale (r) le long de la première hauteur d'élément de guidage (H1, H1', H1") et/ou la deuxième longueur d'élément de guidage (L2, L2', L2") de l'au moins un deuxième élément de guidage (22, 24) varie ou est constante dans la direction radiale (r) le long de la deuxième hauteur d'élément de guidage (H2, H2', H2") ;
et/ou
- la première hauteur d'élément de guidage (H1, H1', H1") de l'au moins un premier élément de guidage (21, 23) et/ou la deuxième hauteur d'élément de guidage (H2, H2', H2") de l'au moins un deuxième élément de guidage (22, 24) varie ou est constante le long de l'axe axial (A) ; et/ou
- la première épaisseur d'élément de guidage (D1, D1', D1") de l'au moins un premier élément de guidage (21, 23) varie ou est constante dans la direction radiale (r) le long de la première hauteur d'élément de guidage (H1, H1', H1") et/ou la deuxième épaisseur d'élément de guidage (D2, D2', D2") de l'au moins un deuxième élément de guidage (22, 24) varie ou est constante dans la direction radiale (r) le long de la première hauteur d'élément de guidage (H2, H2', H2").

14. Dispositif de guidage de flux de gaz (2) selon l'une au moins des revendications précédentes, dans lequel l'unité de guidage (20) est conçue intégralement avec le corps annulaire (10) ou est insérée dans le canal annulaire (13) du corps annulaire (10), dans lequel l'unité de guidage (20) conçue intégralement avec le corps annulaire (10) est réalisée par enlèvement de matière et/ou par coulage et/ou par assemblage.

15. Dispositif de guidage de flux de gaz (2) selon l'une au moins des revendications précédentes, dans lequel le corps annulaire (10) présente une enveloppe extérieure (15) avec un rayon d'enveloppe extérieure (AR), dans lequel l'enveloppe extérieure (15) est conçue de préférence en forme de cylindre.

16. Dispositif de guidage de flux de gaz (2) au moins selon la revendication 3 et la revendication 15, dans lequel le corps annulaire (10) présente une première section d'extrémité de corps annulaire (11') partant de la première extrémité de corps annulaire (11) et une deuxième section d'extrémité de corps annulaire (12') partant de la deuxième extrémité de corps annulaire (12), entre lesquelles s'étend une section intermédiaire de corps annulaire (17), dans lequel la première section d'extrémité de corps annulaire (11') et/ou la deuxième section d'extrémité de corps annulaire (12') présente au moins un évidement ou alésage (16) réparti sur la circonférence, s'étendant de l'enveloppe extérieure (15) à l'enveloppe intérieure (14) du corps annulaire (10).

17. Dispositif de guidage de flux de gaz (2) selon l'une au moins des revendications précédentes, présentant au moins une grille amovible séparément, laquelle est disposée en amont du corps annulaire (10) ou à l'intérieur de celui-ci.

18. Dispositif de guidage de flux de gaz (2) selon la revendication 17, dans lequel au moins une grille amovible séparément est disposée en amont de l'unité de guidage (20) et au moins une grille amovible séparément est disposée en aval de l'unité de guidage (20) dans la direction du flux de gaz.
